# EUROPEAN PATENT SPECIFICATION

(11) **EP 0 747 027 B1**
(45) Date of publication and mention of the grant of the patent: **27.08.2003**
(21) Application number: 96107953.0
(22) Date of filing: 18.05.1996
(51) Int. Cl.: A61F 13/02

(54) **Adhesive tape**
Klebeband
Ruban adhésif

(30) Priority: 06.06.1995 US 468868
(43) Date of publication of application: 11.12.1996
(73) Proprietor: Beiersdorf Aktiengesellschaft, 20245 Hamburg (DE)
(72) Inventor: Knudsen, Ebbe, Charlotte, NC 28277 (US)

(56) References cited:
- WO-A-92/11333
- US-A- 4 024 312
- US-A- 4 402 696
- US-A- 4 807 613
- US-A- 5 213 565

## Description

The invention relates to a pressure-sensitive and stretchable adhesive tape for removal by stretching, especially removal without damage to the substrate or painless to skin.

Tapes which can be removed by stretching are known in the art. US 4,024,312 teaches such a tape having an extensible and elastic backing composed of a block copolymer, which backing is laminated with an adhesive layer. The tape is easily stretchable and may be removed from an application surface by stretching the tape lengthwise in a direction substantially parallel to the surface, which shall make it suitable for medical applications where painless removal is desired. In practical medical application however, that tape has not fulfilled the high expectation because the tape, once adhered to the skin, needs considerable strength for lengthwise stretching, which the skin finds hard to stand, resulting even in pain, which is especially critical with skin covered by injuries or the like. Thus, products of this type have not made it to commercial scale, even though the patent dates from 1977.

Since, several patents were published, disclosing similar type products. **DE 33 31 016 C2** teaches the use of an adhesive foil for adhesive connections which can again be separated, which foil has no backing but consists of adhesive only, which is thermoplastic rubber and tackifying resins. That foil has high elasticity and low plasticity, adhesion is lower than cohesion and adhesiveness disappears largely when stretched. Such lengthwise stretching in the plane of the adhesive connection allows removal of the foil and thereby disconnecting the connected parts without damage. Medical use is not what that invention is for, especially in it's double side adhesive version. However, if such products were used on skin, the same problems would arise as discussed before.

**DE 37 14 453 C1** discloses a blowing piece (1), dynamite or the like, having a double side adhesive tape (4) fixed on the back side of that piece, the other adhesive side of the tape (4) fixed on the back side of that piece, the other adhesive side of that tape (4) being suitable for adhering to a substrate which has to be blown up. In order to separate that piece from the substrate, stretching tape (4) by pulling on a protruding piece (6) of that tape releases the piece (1) undamaged. This, again, is a technical application of such stretchable adhesive tape, with no teaching for any medical use.

**WO 92/11332** describes a removable adhesive tape, pressure sensitive and comprising a highly extensible backing, bearing on at least one major surface thereof a layer of photopolymerized acrylic pressure-sensitive adhesive, capable of being removed by lengthwise stretching. If such tape, which is primarily designated for technical purpose, is applied to skin, the above problems would arise, in view of considerable forces necessary for that stretching.

**WO 92/11333** is for a removable adhesive tape which can be removed from a substrate without damaging that substrate. The tape has a highly extensible and substantially inelastic backing and a layer of pressure-sensitive adhesive thereon. The backing has a high tensile strength, has a lengthwise elongation at break of at least about 150% with less than about 50% elastic recovery after stretching, which stretching again should be lengthwise, substantially parallel to the surface. While technical use is in the focus, nothing in that disclosure would solve the problems in medical applications, as above. **WO 93/01979** teaches such stretchable adhesive tape for use in packages and securing objects together for storage and shipping.

US-A-5,213,565 discloses a tape for attaching bandages, with non-adhesive tabs at each end.

US-A-4,024,312 discloses a pressure-sensitive adhesive tape which comprises a highly extensible and elastic backing film.

**DE 42 22 849 C1** is for a strip of adhesive foil along the disclosure of DE 33 31 016 C2, with one end thereof carrying a tab, covered by a UV-impermeable cover-foil in order to protect the adhesive from UV-damage and at the same time having a tab to grasp and pull for stretching. The application is for technical use, no teaching is for medical applications. So is the teaching of **DE 42 33 872 C2,** which is for a hook having a self-adhesive strip of foil according to the foregoing. A similar hook or support means is disclosed in **WO 94/21157.**

**WO 95/06691** teaches a removable foam adhesive which is quite along the disclosure of WO 92/11333 with an added foam part, and again, for technical use primarily, with no teaching as to the problems which would arise under medical applications, as discussed before.

The prior art, though teaching several self-adhesive tapes releasable by lengthwise stretching, therefore gives no indication what to do for specific applications without harm to the surface to which it is to adhere, especially easily damagable surfaces or skin, in which such a strip of a tape would have to be released by stretching in the plane of the substrate. The damage and pain done to that skin generally would make such products useless, despite release by stretching, and many other surfaces may be damaged if so treated.

The present invention provides an easily removable pressure-sensitive tape, especially a strip of such tape, for medical or other use and for painless and/or without residue release by stretching without damaging the skin or other surface which separates from a surface when stretched lengthwise, especially by loosing its adhesiveness in whole or in part, when stretched lengthwise.

The tape according to the invention has on opposite ends tabs, either on two opposite ends one tab each, i.e. two tabs in total, or on all opposite ends or sides tabs, like in a rectangular tape on all four sides four tabs in total, or in case of a more or less round tape a tab all around the outer edge of the tape. All such tapes can be grasped by two hands on opposite tabs, so as to pull in opposite directions, however substantially parallel to the surface to which the tape adheres. That simultaneous stretching in opposite directions results in a very smooth to skin, painless and easy release of the tape, which is in great contrast to the high adhesiveness of the tape, and which is also in great contrast to the stress and pain given to the surface, skin etc. when removing a similar tape with one tab only.

The materials used for the tape according to the invention are preferably those shown in the prior art cited above, especially DE 33 31 016, WO 92/11332 and WO 92/11333.

The materials used in the medical tape according to the invention are preferably such as are useful for medical use, especially with low irritation to skin, as is well known in the art, but also carriers, tab material, wound dressings etc. are preferably such as are well known in the art for medical tapes.

Preferably, there is a wound dressing placed on part of the adhesive surface of the tape between the tabs, and again preferably that wound dressing is completely surrounded by the adhesive surface.

Tabs can be made by application of e.g. a thin foil or non-adhesive lacquer or the like to the surface of the adhesive. In case a carrier is used, however, that carrier itself can be the tab, i.e. the adhesive is applied to that carrier only partly, (zone coating) so as to leave a side portion of the carrier without adhesive, which results in a tab free of adhesive.

A preferred adhesive used in a tape according to the invention is of the type disclosed in DE 42 22 849 C1, corresponding to US Serial No. 087,622 in which the tape consists essentially of an adhesive foil on the basis of thermoplastic rubber and tackifying resins, in which the foil has high elasticity and low plasticity and in which adhesion is lower than cohesion, and in which the adhesiveness disappears substantially under stretching, and in which the ratio of removal-tearing strength to breaking-strength is at least 1 : 1,5. For further details, reference is made to the text of DE 42 22 849 C1, including the examples of that patent. The tab material can be a thin foil, especially a UV-impermeable foil, such as a PETP (Polyethyleneterephthalate) foil. The back side of the medical tape is preferably non-adhesive, e.g. by a non-adhesive print on that back side, or by a thin layer of a non-woven, which does not interfere with stretching the tape and at the same time gives a convenient feeling to the back side of the tape. Or, a highly extensible film can be laminated to the back side, preferably a highly extensible but substantially inelastic film, e.g. described in WO 92/11333, corresponding to US Serial No. 632,173, especially linear low density Polyethylene film (available from consolidated Thermoplastics Co.) as backing material.

Another preferred type of adhesive tape has a highly extensible and substantially inelastic backing film and a layer of pressure sensitive adhesive on one side thereof. Preferably, in such tape the backing has a high tensile strength, has a lengthwise elongation to break of at least about 150% with less than about 50% elastic recovery after stretching. For further details, reference is made to WO92/11333, including the examples of that patent application. Tabs may again consist of a thin foil, as above, and the back side is preferably non-adhesive, preferably by the backing used. For more details, especially for adhesives to be used and other materials to be incorporated, reference is made to WO 92/11332 and WO 94/21157. Acrylate adhesives are most preferred, in view of their low skin irritation potential.

Another preferred tape according to the invention is one in which the tape has a highly extensible and elastic backing film and a layer of pressure sensitive adhesive on one side thereof, especially one in which the backing film has a lengthwise elongation to break of at least about 200%, and a 50% rubber modulus of not above about 2000 lbs./sq.inch. For further details, reference is made to US 4.024.312, including the examples of that patent. The tabs again can be of a thin foil, as above, and the back side preferably is non-adhesive due to the backing film used.

The invention also teaches a method of easy removal, especially painless removal from skin of a tape as described herein, in which a tape applied is grasped on its tabs by hands and stretched so that the tabs are separated one from another, i.e. stretched in opposite directions, but substantially parallel to the surface of the substrate, like the skin to which it adheres. Such method is especially useful for releasing a highly adhesive tape without any pain or damage to the substrate or skin and in a very smooth and gentle way, especially on problematic or freshly healed skin or the like difficult substrates.

Further details and embodiments of the invention will be shown and explained with reference to the drawings, in which
- Fig. 1: is a schematic perspective view of a medical tape according to the invention;
- Fig. 2: is a side view of the medical tape according to Fig. 1;
- Fig. 3: is a schematic perspective view of another medical tape according to the invention;
- Fig. 4: shows the medical tape of Fig. 1 applied to a leg; and
- Fig. 5: shows a schematic perspective view of still another medical tape according to the invention.

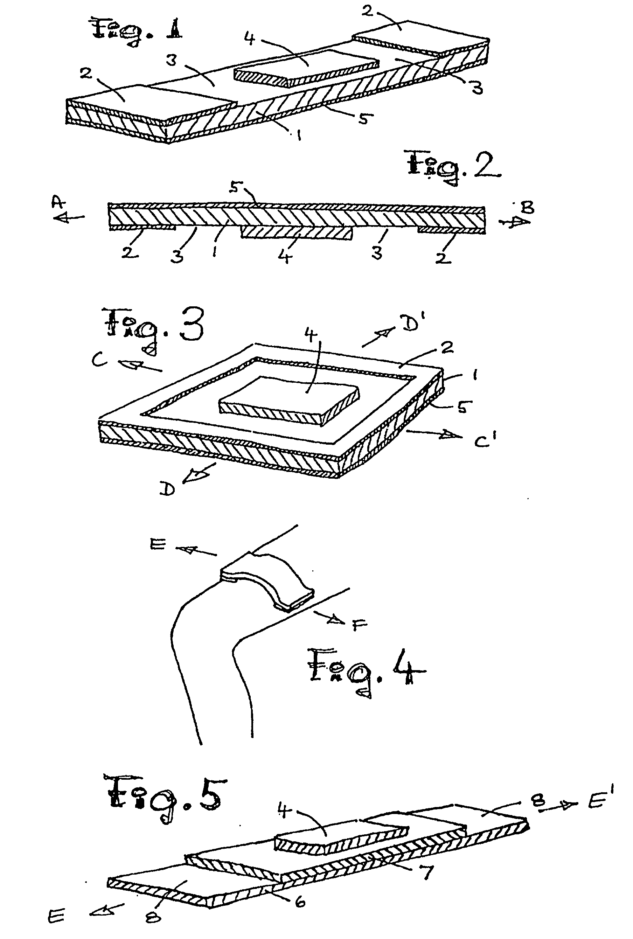

In a medical tape as shown in Fig. 1, an adhesive as described in Example 2 of DE 42 22 849 C1 of 200 µm thickness is identified as 1. Parts of the adhesive surface at both ends of the medical tape are covered by tabs 2, consisting of a PETP foil of 25 µm thickness, whereas other parts of the adhesive surface 3 are open for being adhesively adhered to a skin surface. Optionally and preferably, there is a wound dressing 4 on that adhesive surface, which is a non-woven, containing and antibacterial agent and which has a surface non-adhering to a wound. Alternatively, similar materials can be used as wound dressing, as is known in the art, especially absorptive materials, such as wovens, fleece, foams or gels. The back side of the medical tape is made non-adhesive by a lacquer 5 printed on that surface. That non-adhesive layer 5 can, however, be also a thin layer of non-woven, as widely used in medical applications. In Fig. 2, a side view of the medical tape of Fig. 1 two arrows A and B indicate the direction in which the tape has to be stretched when release from a surface, like skin, is to be effected.

Another embodiment of the invention is shown in Fig. 3, again with a 200 µm thick adhesive 1 as according to the Fig. 1 embodiment, and also with respect to other materials used for tabs 2, wound dressing 4 and non-adhesive backside layer 5 reference is made to the Fig. 1 description. However, the tab 2 extends all around the edge portion of the medical tape, so as to allow the tape to be grasped wherever desired, and arrows show how that tape may be released by stretching, such as by arrows C and C' (in opposite directions) or D and D' (also in opposite directions).

In Fig. 4 there is shown a medical tape as per Fig. 1, applied to the skin of a leg. By stretching that tape in the opposite directions of arrows E and F, easy removal can be achieved, just by grasping the two ends / tabs of the tape and with both hands simultaneously and gently pulling the tape apart, as by arrows E and F.

In a medical tape as shown in Fig. 5, a corona treated 50 µm thick foil linear low density polyethylene (consolidated Thermoplastics Co), elastic modulus 28,986 (psi), elongation 748 % and recovery 5,4 %, carries a layer 7 of a UV-crosslinked acrylate adhesive. On both ends of the tape, tabs 8 remain adhesive-free, and on the adhesive layer 7 there is placed a wound dressing 4, as in Fig. 1. By pulling and stretching the tape at both tabs 8 in opposite directions, as indicated by arrows E and E', the tape can be released from a surface, especially from skin.

## Claims

1. A pressure-sensitive and stretchable adhesive tape, which separates from a substrate when stretched lengthwise in a direction substantially parallel to a surface to which is adhered, **characterized in that** the tape has at least two spaced tabs with non-adhesive surfaces and an adhesive, which adhesion is lower than cohesion and which adhesiveness disappears under stretching.

2. Tape according to claim 1, in which a wound dressing is placed on part of the adhesive surface of the tape between the tabs.

3. Tape according to claim 2, in which the wound dressing is surrounded by the adhesive surface.

4. Tape according to claim 1, in which the tape is substantially rectangular and has tabs on all four sides.

5. Tape according to claim 1, in which the back side of the tape opposite to the pressure-sensitive adhesive is non-adhesive.

6. Tape according to claim 1, in which the tape consists essentially of an adhesive foil on the basis of thermoplastic rubber and tackifying resins, in which the foil has high elasticity and low plasticity and in which the ratio of removal-tearing strength to breaking-strength is at least 1:1,5.

7. Tape according to claim 1, in which the tape has a backing film with a lengthwise elongation to break of at least about 150% with less than about 50% elastic recovery after stretching and a layer of pressure sensitive adhesive on one side thereof.

8. Tape according to claim 1, in which the tape has a highly extensible and elastic backing film and a layer of pressure sensitive adhesive on one side thereof.

9. Tape according to claim 9, in which the backing film has a lengthwise elongation to break of at least about 200%, and a 50% rubber modulus of not above 13.79·10⁶ N/m³ (2000 lbs./sq.inch).

## Patentansprüche

1. Dehnbares Haftklebeband für Verklebungen, die durch längsgerichtetes Ziehen im wesentlichen in der Verklebungsebene wieder von einem Substrat lösbar sind, **dadurch gekennzeichnet, daß** das Band über mindestens zwei voneinander beabstandete Anfasser mit nicht klebeaktiven Oberflächen und einen Klebstoff verfügt, wobei die Adhäsion geringer als die Kohäsion ist und das Haftvermögen beim Dehnen verschwindet.

2. Haftklebeband nach Anspruch 1, bei dem auf Teil der klebeaktiven Bandoberfläche zwischen den Anfassern eine Wundauflage angeordnet ist.

3. Haftklebeband nach Anspruch 2, bei dem die Wundauflage von der klebeaktiven Oberfläche umgeben ist.

4. Haftklebeband nach Anspruch 1, bei dem das Haftklebeband im wesentlichen rechteckig ist und auf allen vier Seiten über Anfasser verfügt.

5. Haftklebeband nach Anspruch 1, bei dem die von dem Haftklebstoff abgewandte Rückseite des Bandes nicht klebeaktiv eingestellt ist.

6. Haftklebeband nach Anspruch 1, bei dem das Haftklebeband im wesentlichen aus einer Klebfolie auf Basis von thermoplastischem Kautschuk und klebrigmachenden Harzen besteht, wobei die Klebfolie hohe Elastizität und geringe Plastizität aufweist und das Verhältnis von Abzugskraft zu Reißlast mindestens 1:1,5 ist.

7. Haftklebeband nach Anspruch 1, bei dem das Haftklebeband eine Trägerfolie mit einer Reißdehnung in Längsrichtung von mindestens etwa 150% bei einem Rückstellvermögen von weniger als etwa 50% sowie eine einseitig darauf angeordnete Haftklebstoffschicht aufweist.

8. Haftklebeband nach Anspruch 1, bei dem das Haftklebeband eine elastisch hochdehnbare Trägerfolie und eine einseitig darauf angeordnete Haftklebstoffschicht aufweist.

9. Haftklebeband nach Anspruch 9, bei dem die Trägerfolie eine Reißdehnung in Längsrichtung von mindestens etwa 200% und einen 50%igen Gummi-Modul von höchstens 13,79x10⁶ N/m³ aufweist.

## Revendications

1. Ruban adhésif sensible à la pression et étirable, qui se sépare d'un substrat lorsqu'il est étiré dans le sens de la longueur dans une direction essentiellement parallèle à la surface sur laquelle il adhère, **caractérisé en ce que** le ruban possède au moins deux pattes distantes avec des surfaces non adhésives et un adhésif dont l'adhérence est plus faible que la cohésion et dont l'adhésivité disparaît sous l'effet d'un étirement.

2. Ruban selon la revendication 1, dans lequel un pansement est placé sur la partie de la surface adhésive du ruban entre les pattes.

3. Ruban selon la revendication 2, dans lequel le pansement est entouré d'une surface adhésive.

4. Ruban selon la revendication 1, dans lequel le ruban est essentiellement rectangulaire et possède des pattes sur les quatre côtés.

5. Ruban selon la revendication 1, dans lequel l'envers du ruban à l'opposé de l'adhésif sensible à la pression est non adhésif.

6. Ruban selon la revendication 1, dans lequel le ruban se compose essentiellement d'une feuille d'adhésif à base de caoutchouc thermoplastique et de résines d'adhésivité, dans lequel la feuille a une forte élasticité et une faible plasticité et dans lequel le rapport entre la résistance à la déchirure au retrait et la résistance à la rupture est d'au moins 1:1,5.

7. Ruban selon la revendication 1, dans lequel le ruban possède un film de support possédant un allongement à la rupture dans le sens de la longueur d'au moins environ 150%, avec moins d'environ 50% de reprise élastique après étirement, et une couche d'adhésif sensible à la pression sur une de ses faces.

8. Ruban selon la revendication 1, dans lequel le ruban possède un film de support fortement extensible et élastique et une couche d'adhésif sensible à la pression sur une de ses faces.

9. Ruban selon la revendication 9, dans lequel le film de support possède un allongement à la rupture dans le sens de la longueur d'au moins environ 200% et un module caoutchouteux à 50% non supérieur à 13,79.10⁶ N/m³ (2 000 Ibs/in²).
